# EUROPEAN PATENT SPECIFICATION

(11) **EP 3 308 802 B1**
(45) Date of publication and mention of the grant of the patent: **19.08.2020**
(21) Application number: 16807715.4
(22) Date of filing: 19.05.2016
(51) Int. Cl.: A61K 49/00, A61K 49/18

(54) **HYDROPHILIC PARTICLES, METHOD FOR PRODUCING SAME, AND CONTRAST AGENT UTILIZING SAME**
HYDROPHILE PARTIKEL, VERFAHREN ZUR HERSTELLUNG DAVON UND KONTRASTMITTEL MIT VERWENDUNG DAVON
PARTICULES HYDROPHILES, PROCÉDÉ DE PRODUCTION DE CELLES-CI, ET AGENT DE CONTRASTE UTILISANT CELLES-CI

(30) Priority: 10.06.2015 KR 20150081804
(43) Date of publication of application: 18.04.2018
(73) Proprietor: Korea Basic Science Institute, Cheongju-si, Chungcheongbuk-do 28119 (KR); Korea Research Institute of Chemical Technology, Daejeon 34114 (KR)
(72) Inventor: HONG, Kwan Soo, Cheongju-si Chungcheongbuk-do 28123 (KR); PARK, Hye Sun, Sejong 30130 (KR); NAM, Sang Hwan, Sejong 30130 (KR); SUH, Yung Doug, Seoul 08016 (KR)
(74) Representative: Wallace, Sheila Jane
(86) International application number: PCT/KR2016/005323
(87) International publication number: WO 2016/200074

(56) References cited:
- KR-A- 20090 004 374
- KR-A- 20100 007 665
- KR-A- 20120 017 556
- MA YAN ET AL: "Indocyanine green loaded SPIO nanoparticles with phospholipid-PEG coating for dual-modal imaging and photothermal therapy", BIOMATERIALS, vol. 34, no. 31, 17 July 2013 (2013-07-17) , pages 7706-7714, XP028683841, ISSN: 0142-9612, DOI: 10.1016/J.BIOMATERIALS.2013.07.007
- KATARZYNA WYBIERALSKA ET AL: "Removal of organic dyes from aqueous solutions with surfactant-modified magnetic nanoparticles", POLISH JOURNAL OF CHEMICAL TECHNOLOGY, vol. 16, no. 2, 26 June 2014 (2014-06-26), pages 27-30, XP055527690, DOI: 10.2478/pjct-2014-0025
- JAT SANJEEV K ET AL: "A novel aqueous colloidal magnetic nanofluid: removal of surface-adsorbed dye from PDMS surface", MICROFLUIDICS AND NANOFLUIDICS, SPRINGER, DE, vol. 20, no. 1, 5 January 2016 (2016-01-05), pages 1-9, XP035897000, ISSN: 1613-4982, DOI: 10.1007/S10404-015-1670-5 [retrieved on 2016-01-05]
- VERENA MUHR ET AL: "Upconversion Nanoparticles: From Hydrophobic to Hydrophilic Surfaces", ACCOUNTS OF CHEMICAL RESEARCH., vol. 47, no. 12, 27 October 2014 (2014-10-27), pages 3481-3493, XP055528117, US ISSN: 0001-4842, DOI: 10.1021/ar500253g
- ZHENGQUAN LI ET AL: "Multicolor Core/Shell-Structured Upconversion Fluorescent Nanoparticles", ADVANCED MATERIALS, vol. 20, no. 24, 17 December 2008 (2008-12-17), pages 4765-4769, XP055528185, DE ISSN: 0935-9648, DOI: 10.1002/adma.200801056
- MERIAN, J. ET AL.: 'Fluorescent Nanoprobes Dedicated to in Vivo Imaging: From Preclinical Validations to Clinical Translation' MOLECULES vol. 17, no. 5, 2012, pages 5564 - 5591, XP055334975
- WOLFBEIS, O. S.: 'An Overview of Nanoparticles Commonly Used in Fluorescent Bioimaging' CHEM. SOC. REV. vol. 44, no. 14, 26 January 2015, pages 4743 - 4768, XP055334977

## Description

### TECHNICAL FIELD

The present invention disclosed herein relates to a hydrophilic particle, the phase of which is converted by using an amphiphilic organic dye, a method for manufacturing the same, and a contrasting agent using the same.

### BACKGROUND ART

Nanoparticles have been extensively studied for scientific interest and potential applications due to the unique electrical, magnetic, and optical properties, and various functionalities thereof. The application of nanoparticles to the biomedical field have been attracting considerable attention thereto since nanoparticles are expected to improve medical diagnosis and treatment.

For the practical application of nanoparticles to the biomedical field, nanoparticles having both magnetic and fluorescence properties are needed in vivo in vitro applications. From this point of view, studies on multilayer nanoparticles combining magnetic nanoparticles and organic/inorganic phosphors are actively conducted. As a magnetic nanoparticle, a gadolinium nanoparticle which is a paramagnetic material is currently widely used clinically, and an iron oxide-based nanoparticle which is a superparamagnetic material is known to be able to be used as a contrasting agent using MRI.

However, materials constituting the core of such multilayer nanoparticles are mostly heavy metals, and thus, for biomedical applications, a processing for modifying the surface of a nanoparticle is needed. For example, a method of increasing the biocompatibility by introducing a silica layer on the surface of the nanoparticle is representative.

Ma Yan et al., Biomaterials, vol. 34, no. 31, pages 7706-7714 relates to indocyanine green loaded SPIO nanoparticles with phospholipid-PEG coating for dual-modal imaging and photothermal therapy.

Verena Muhr, et al., Accounts of Chemical Research, vol. 47, no. 12, pages 3481-3493 relates to upconversion nanoparticles.

Zhengquan Li, et al., Advanced Materials, vol. 20, no. 24, pages 4765-4769 relates to multicolour core/shell-structured upconversion fluorescent nanoparticles.

### DISCLOSURE OF THE INVENTION

### TECHNICAL PROBLEM

The present invention provides a hydrophilic particle using an amphiphilic organic dye without surface modification.

The present invention also provides a method for manufacturing a hydrophilic particle, the method including a phase conversion method using an amphiphilic organic dye as an interface material.

The present invention also provides a contrasting agent including the hydrophilic particle.

### TECHNICAL SOLUTION

The invention is defined by the claims. Any subject-matter falling outside the scope of the claims is provided for information purposes only.

A hydrophilic particle according to the inventive concept includes a hydrophobic particle, and an amphiphilic organic dye directly absorbed on a surface of the hydrophobic particle. In this case, the hydrophobic particle includes a center particle, and a hydrophobic ligand covering a surface of the center particle, and the amphiphilic organic dye is combined with the hydrophobic ligand by a hydrophobic interaction. The hydrophilic particle has a surface zeta potential lower than a surface zeta potential of the amphiphilic organic dye.

The center particle of the invention includes a transition metal oxide or is an up-conversion particle, and the hydrophobic ligand includes a fatty acid.

In an embodiment, the transition metal oxide may be selected from the group consisting of iron oxide, manganese oxide, titanium oxide, nickel oxide, cobalt oxide, zinc oxide, ceria, and gadolinium oxide.

In an embodiment, the fatty acid may be selected from the group consisting of oleic acid, laurate acid, palmitic acid, linoleic acid, and stearic acid.

In an embodiment, the up-conversion particle may be selected from the group consisting of NaYF₄:Yb³⁺,Er³⁺, NaYF₄:Yb³⁺,Tm³⁺, NaGdF₄:Yb³⁺,Er³⁺, NaGdF₄:Yb³⁺,Tm³⁺, NaYF₄:Yb³⁺,Er³⁺/NaGdF₄, NaYF₄:Yb³⁺,Tm³⁺/NaGdF₄, NaGdF₄:Yb³⁺,Tm³⁺/NaGdF₄, and NaGdF₄:Yb³⁺,Er³⁺/NaGdF₄.

The amphiphilic organic dye of the invention is selected from the group consisting of cyanine.

The hydrophilic particle of the invention is obtainable from a process for manufacturing a hydrophilic particle comprising the steps of:
preparing the above mentioned hydrophobic particle dispersed in an organic phase; and
mixing the above mentioned hydrophobic particle in the organic phase with the above mentioned amphiphilic organic dye in an aqueous phase to form the hydrophilic particle of the invention;
wherein the amphiphilic organic dye is directly adsorbed on a surface of the hydrophobic particle to phase-convert the hydrophobic particle to the hydrophilic particle dispersed in the aqueous phase.

In an embodiment, the surface zeta potential of the amphiphilic organic dye may be a value measured when the amphiphilic organic dye is present alone.

In an embodiment, the surface zeta potential of the hydrophilic particle may be a negative charge.

In an embodiment, an average diameter of the hydrophilic particle may be greater than an average diameter of the hydrophobic particle.

A method for manufacturing a hydrophilic particle according to another inventive concept includes preparing a hydrophobic particle dispersed in an organic phase, and mixing the hydrophobic particle in the organic phase with an amphiphilic organic dye in an aqueous phase to form a hydrophilic particle. In this case, the amphiphilic organic dye is directly absorbed on a surface of the hydrophobic particle to phase-convert the hydrophobic particle to the hydrophilic particle dispersed in the aqueous phase The centre particle, hydrophobic particle and the amphiphilic organic dye of the invention are those mentioned in the claims.

In an embodiment, the mixing of the hydrophobic particle and the amphiphilic organic dye may include adding the hydrophobic particle in the organic phase to the amphiphilic organic dye in the aqueous phase, and ultrasonicating the mixture of the hydrophobic particle and the amphiphilic organic dye to form a water-in-oil (O/W) emulsion.

In an embodiment, the organic phase may include an organic solvent selected from the group consisting of chloroform, cyclohexane, hexane, heptane, octane, isooctane, nonane, decane, and toluene.

In the present invention, the hydrophobic particle includes a hydrophobic ligand on a surface thereof, and the amphiphilic organic dye is combined with the hydrophobic ligand by a hydrophobic interaction.

In an embodiment, the method may further include, after forming the hydrophilic particle, evaporating the organic solvent constituting the organic phase.

A contrasting agent according to another inventive concept includes a hydrophilic particle according to the inventive concept. As indicated above, the hydrophilic particle includes a hydrophobic particle, and an amphiphilic organic dye directly adsorbed on a surface of the hydrophobic particle. A surface zeta potential of the hydrophilic particle is lower than a surface zeta potential of the amphiphilic organic dye.

In an embodiment, the contrasting agent may be used for magnetic resonance imaging, optical imaging, or magnetic resonance imaging and optical imaging.

### ADVANTAGEOUS EFFECTS

A hydrophilic particle according to the inventive concept may have two contrasting functions through the combination of an amphiphilic organic dye positioned on the surface thereof, and a center particle. In addition, a hydrophilic particle according to the inventive concept has high biocompatibility and may increase the stability of an organic dye combined to the surface thereof. Furthermore, a method for manufacturing a hydrophilic particle according to the inventive concept may be performed simply and quickly through a phase conversion method without surface modification of a particle and a surfactant.

### BRIEF DESCRIPTION OF THE DRAWINGS

FIG. 1A is a cross-sectional view schematically showing a hydrophilic particle according to embodiments of the inventive concept. FIG. 1B is an enlarged cross-sectional view of the region M of FIG. 1A;
FIG. 2 is a cross-sectional view schematically showing a method for manufacturing a hydrophilic particle according to embodiments of the inventive concept;
FIG. 3 shows a TEM image and a size analysis result of iron oxide nanoparticles (IONP) dispersed in an organic solvent;
FIG. 4 shows the process of purification using the magnetic force of an iron oxide nanoparticle dispersed in an aqueous phase;
FIG. 5 shows a TEM image and a size analysis result of indocyanine green coated iron oxide nanoparticles (ICG coated IONP) dispersed in an aqueous phase;
FIG. 6 is a comparative analysis result of the surface charges of an iron oxide nanoparticle coated with indocyanine green (IONP-ICG) and dispersed in an aqueous phase, and a pure indocyanine green (ICG) solution;
FIG. 7 is a FT-IR analysis result of indocyanine green (ICG), an iron oxide nanoparticle (IONP), and an iron oxide nanoparticle coated with indocyanine green (IONP-ICG);
FIGS. 8 and 9 respectively show a comparison analysis result of the absorbance spectrum and fluorescence spectrum of an iron oxide nanoparticle coated with indocyanine green (IONP-ICG) and dispersed in an aqueous phase, and a pure indocyanine green (ICG) solution;
FIG. 10 shows T2-weighted MR phantom images and r2 values in accordance with various concentrations of an iron oxide nanoparticle coated with indocyanine green dispersed in an aqueous phase;
FIG. 11 are an image of fluorescence signal and an MR image thereof, the signal appearing at the lymph node after the injection of iron oxide nanoparticles coated with indocyanine green into a sole a mouse;
FIG. 12 shows a TEM image and a size analysis result of up-conversion nanoparticles dispersed in an organic solvent;
FIG. 13 are photoluminescence (PL) images (UCNP; a, c) of an up-conversion nanoparticle coated with indocyanine green, and fluorescence images (ICG; b, d) of an up-conversion nanoparticle coated with indocyanine green;
FIG. 14 is a single particle fluorescence image showing the optical stability of an up-conversion nanoparticle coated with indocyanine green.

### MODE FOR CARRYING OUT THE INVENTION

Objects, other objects, features, and advantages of the inventive concept described above may be understood easily by reference to the exemplary embodiments and the accompanying drawings.

In the accompanying drawings, the sizes, thicknesses, and the like of the structures are exaggerated for clarity of the inventive concept. Also, it will be understood that, although the terms first, second, etc. may be used herein to describe various elements, these elements should not be limited by these terms. These terms are only used to distinguish one element from another. Each embodiment described and exemplified herein also includes the complementary embodiment thereof. The term "and/or," is used herein to include at least one of the elements listed before and after. Like reference numerals refer to like elements through the specification.

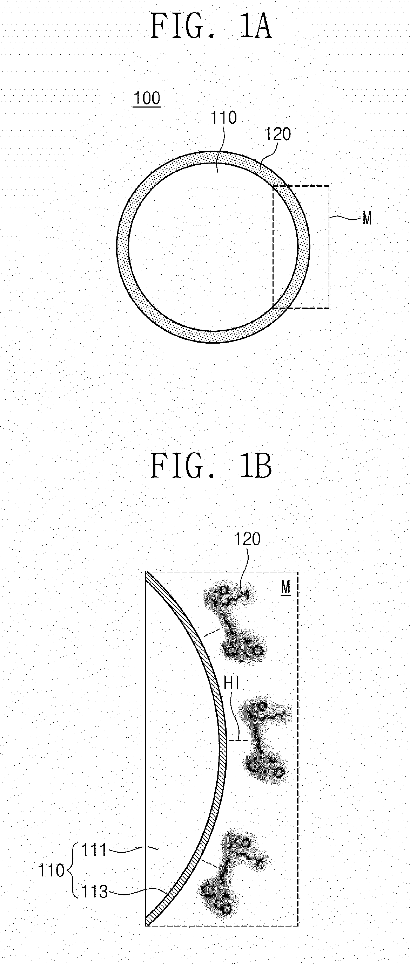

FIG. 1A is a cross-sectional view schematically showing a hydrophilic particle according to embodiments of the inventive concept. FIG. 1B is an enlarged cross-sectional view of the region M of FIG. 1A.

Referring to FIGS. 1A and 1B, a hydrophilic particle 100 including a hydrophobic particle 110 and an amphiphilic organic dye 120 is provided. The amphiphilic organic dye 120 is directly adsorbed on the surface of the hydrophobic particle 110. The adsorption may be caused by the physical interaction between the hydrophobic particle 110 and the amphiphilic organic dye 120.

The hydrophobic particle 110 includes a center particle 111, and a hydrophobic ligand 113 coated on the surface of the center particle 111. For example, the hydrophobic ligand 113 may form a single layer and cover the surface of the center particle 111. As another example, the hydrophobic ligand 113 may be uniformly or non-uniformly combined to the inside and to the surface of the center particle 111. The hydrophobic ligand 113 may impart hydrophobicity to the central particle 111. Thus, a plurality of hydrophobic particles 110 may be solely dispersed in an organic solvent without surfactant.

According to an embodiment of the inventive concept, the center particle 111 may be a transition metal oxide particle. That is, the particle center 111 may include one or more transition metal oxides selected from the group consisting of iron oxide, manganese oxide, titanium oxide, nickel oxide, cobalt oxide, zinc oxide, ceria and gadolinium oxide. The transition metal oxide, especially iron oxide, may have magnetic properties under an external magnetic field. When the external magnetic field is removed, the magnetism remaining in the transition metal oxide may disappear. Therefore, side effects due to the remaining magnetism may be reduced. Furthermore, the transition metal oxide may be biodegraded in the body, so that the biocompatibility thereof may be excellent. The transition metal oxide may be used as a cell marking material for magnetic resonance imaging (MRI) tracking of therapeutic cells.

In another embodiment, the center particle 111 may be an up-conversion particle. The up-conversion particle may be a particle capable of emitting a visible ray when a near infrared ray is irradiated thereon. The up-conversion particle may be a particle which is an inorganic host doped with a rare earth element. For example, the up-conversion particle may include one or more selected from the group consisting of NaYF₄:Y b³⁺,Er³⁺, NaYF₄:Yb³,Tm³⁺, NaGdF₄:Yb³⁺,Er³⁺, NaGdF₄:Yb³⁺,Tm³⁺, NaYF₄:Yb³⁺,Er³⁺/NaGdF₄, NaYF₄:Yb³⁺,Tm³⁺/NaGdF₄, NaGdF₄:Yb³⁺,Tm³⁺/NaGdF₄, and NaGdF₄:Yb³⁺,Er³⁺/NaGdF₄.

Furthermore, since the surface thereof exhibits hydrophobicity, the hydrophobic particle 110 is not particularly limited as long as the particle may be dispersed in the organic solvent.

The hydrophobic ligand 113 includes a fatty acid. For example, the fatty acid may include at least one selected from the group consisting of oleic acid, lauric acid, palmitic acid, linoleic acid, and stearic acid.

The amphiphilic organic dye 120 is selected from the group consisting of cyanine. For example, the amphiphilic organic dye 120 may include indocyanine green.

By a hydrophobic interaction (HI) between a hydrophobic group of the amphiphilic organic dye 120 and the hydrophobic ligand 113, the amphiphilic organic dye 120 is combined with the hydrophobic ligand 113. Thus, as described above, the amphiphilic organic dye 120 is directly adsorbed (or coated) on the surface of the hydrophobic particle 110.

The average diameter of the hydrophilic particle 100 may be 10 nm to 1000 nm. In this case, the average diameter of the hydrophilic particle 100 may be greater than the average diameter of the hydrophobic particle 110. In an embodiment of the inventive concept, the average diameter of the hydrophilic particle 100 may be greater than two times the average diameter of the hydrophobic particle 110.

The hydrophilic particle 100 may have a first surface zeta potential. When the amphiphilic organic dye 120 is present alone, the amphiphilic organic dye 120 may have a second surface zeta potential. In this case, the first surface zeta potential is lower than the second surface zeta potential. That is, the hydrophilic particle 100 may have relatively negative charge properties compared to the amphiphilic organic dye 120 present alone. For example, the first surface zeta potential may be a negative charge, and may specifically be -100 mV to -10 mV.

As the amphiphilic organic dye 120 is adsorbed on the surface of the hydrophobic particle 110, the hydrophilic group of the amphiphilic organic dye 120 may be exposed to the outside more than the hydrophobic group. Accordingly, the hydrophilic group of the amphiphilic organic dye 120 may be relatively more distributed on the surface of the hydrophilic particle 100. Thus, the surface charge of the hydrophilic particle 100 may have relatively a negative value compared to the amphiphilic organic dye 120 present alone.

The hydrophilic particle 100 may be used as a contrasting agent through the amphiphilic organic dye 120 positioned on the surface thereof. In one embodiment, when the amphiphilic organic dye 120 is a fluorescence organic dye, fluorescence contrasting may be possible for the hydrophilic particle 100. Furthermore, the center particle 111 in the hydrophilic particle 100 may have a contrasting function. For example, when the center particle 111 includes a transition metal oxide, MR contrasting may be possible. In this case, the hydrophilic particle 100 may have two contrasting functions (MR contrasting and fluorescence contrasting), and therefore, may be utilized for in vivo and in vitro molecular imaging in the biomedical field. As another example, when the center particle 111 is an up-conversion particle, photoluminescence (PL) contrasting may be possible. In this case, the hydrophilic particle 100 may have two contrasting functions (PL contrasting and fluorescence contrasting).

In addition, since the hydrophilic particle 100 has hydrophilicity, the biocompatibility thereof may be excellent. Since the amphiphilic organic dye 120 is combined with the hydrophobic particle 110 by a hydrophobic interaction (HI), the fluorescence stability of the amphiphilic organic dye 120 may be increased.

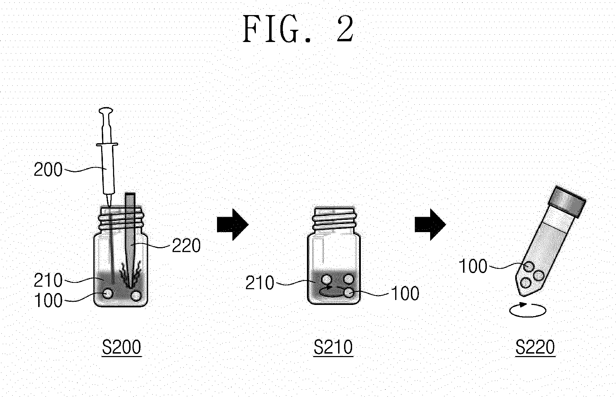

FIG. 2 is a cross-sectional view schematically showing a method for manufacturing a hydrophilic particle according to embodiments of the inventive concept.

Referring FIGS. 1A, 1B, and 2, by ultrasonicating a mixture of a first solution 200 and a second solution 210, an emulsion 220 may be formed S200. Specifically, first, the first solution 200 may be prepared. The first solution 200 includes the hydrophobic particles 110, and an organic solvent in which the hydrophobic particles 110 are dispersed. In other words, the hydrophobic particles 110 are dispersed in an organic phase. Preparing the first solution 200 may include using various known methods such as a coprecipitation method, a thermal decomposition method, a hydrothermal synthesis method, or a microemulsion method. For example, the first solution 200 may be prepared by using the thermal decomposition method. When the thermal decomposition method is used, the fine size adjustment of the hydrophobic particles 110 is possible, and the size distribution of the hydrophobic particles 110 may be uniform, and the crystallinity of the hydrophobic particles 110 may be increased.

Each of the hydrophobic particles 110 include a center particle 111, and a hydrophobic ligand 113 coated on the surface of the central particle 111. In one embodiment, the center particle 111 may be a transition metal oxide particle, and in another embodiment, the center particle 111 may be an up-conversion particle. However, the hydrophobic particle 110 is not particularly limited as long as the surface of the particle exhibits hydrophobicity, so that the particle may be dispersed in the organic solvent. The detailed description of the hydrophobic particle 110 may be the same as described above with reference to FIG. 1A and FIG. 1B. The organic solvent may be one or more selected from the group consisting of chloroform, cyclohexane, hexane, heptane, octane, isooctane, nonane, decane, and toluene, and is not particularly limited.

The second solution 210 may be prepared. The second solution 210 may be prepared by mixing the amphiphilic organic dye 120 and water. In other words, the amphiphilic organic dye 120 is an aqueous phase. For example, the amphiphilic organic dye 120 may be a fluorescence organic dye having both a hydrophilic group and a hydrophobic group in the molecule thereof. The detailed description of the amphiphilic organic dye 120 may be the same as described above with reference to FIG. 1A and FIG. 1B.

The mixture may be prepared by adding the first solution 200 to the second solution 210. By using an ultrasonic tip apparatus, ultrasonicating of the mixture may be performed. The ultrasonicating may be performed for 10 seconds to 10 minutes. Thus, the first solution 200 and the second solution 210 is homogeneously mixed to form the oil-in-water (O/W) emulsion 220.

At the same time as the first solution 200 and the second solution 210 are mixed, the amphiphilic organic dye 120 may be directly adsorbed on the surface of the hydrophobic particle 110. Thus, the hydrophilic particle 100 which is the hydrophobic particle 110 coated with the amphiphilic organic dye 120 on the surface thereof may be formed. Specifically, by a hydrophobic interaction (HI) between a hydrophobic group of the amphiphilic organic dye 120 and the hydrophobic ligand 113, the amphiphilic organic dye 120 may be combined with the hydrophobic ligand 113. Without chemical reaction and only by ultrasonicating, the amphiphilic organic dye 120 may be directly adsorbed on the surface of the hydrophobic particle 110 by a physical interaction between the amphiphilic organic dye 120 and the hydrophobic particle 110.

By stirring the emulsion 220, the organic solvent may be evaporated S210. The stirring may be performed for 1 minute to 60 minutes. As a result, the hydrophilic particles 100 may be dispersed in an aqueous phase. Specifically, the hydrophilic particles 100 may be stably dispersed in an aqueous phase due to a hydrophilic group of the amphiphilic organic dye 120 present on the surface thereof. In other words, without surfactant, the phase of the hydrophobic particle 110 may be converted through the amphiphilic organic dye 120.

Next, the hydrophilic particles 100 may be purified S220. Performing the purification may include using centrifugation or using a magnetic force. For example, using centrifugation may include performing centrifugation on the emulsion 220, and removing supernatant. This process may be repeatedly performed until the amphiphilic organic dye 120 which is dispersed without being absorbed on the surface of the hydrophilic particle 100 is removed.

As another example, when the center particle 111 of the hydrophilic particle 100 is a transition metal oxide particle, the magnetic force may be used. Using the magnetic force may include adhering a powerful magnet to the emulsion 220, and then removing supernatant. This process may be repeatedly performed until the amphiphilic organic dye 120 which is dispersed without being absorbed on the surface of the hydrophilic particle 100 is removed.

A method for manufacturing the hydrophilic particle 100 according to the inventive concept may be performed simply and quickly by a phase conversion method using the amphiphilic organic dye 120 as an interface material, without surface modification of a particle, or surfactant.

The hydrophobic particle, amphiphilic organic dye and interaction of the present invention are those defined in the claims.

Hereinafter, preferred experimental examples will be described in order to facilitate understanding of the inventive concept. It should be understood, however, that the following experimental examples are for illustrative purposes only and are not intended to limit the scope of the inventive concept.

### Experimental Example 1: Synthesis of an iron oxide nanoparticle

36 g of iron-oleate (Fe-oleate, 40 mmol), 5.7 g of oleic acid (oleic acid, 20 mmol), and 200 g of octadecene (1-octadecene) were mixed. The mixture was stirred for 30 minutes under reduced pressure at room temperature to remove gas and water in the mixture. The mixture was heated to 320°C at a heating rate of 3.3°C/min. At this time, the reduced pressure state was maintained up to 200°C, and at a temperature higher than 200°C, an inert atmosphere was maintained. After the mixture was stirred for 30 minutes at 320°C, a heater was removed, and the mixture was slowly cooled to room temperature. Thereafter, ethanol was added to the mixture in six times the volume of the mixture to precipitate the formed iron oxide nanoparticle. Then, supernatant was removed, and the iron oxide nanoparticle was separated to be redispersed in n-hexane. The concentration of the iron oxide nanoparticle in n-hexane was adjusted to be 10 mg/ml (Example 1).

The nanoparticle of Example 1 was dropped on a copper grid coated with carbon to prepare a sample, and a transmission electron microscope (TEM) image thereof was obtained with a high resolution electron microscope of 200 kV (Tecnai F20). Furthermore, the size of the nanoparticle of Example 1 was measured using a particle size surface charge analyzer (Zetasizer Nano-ZS, Otsuka).

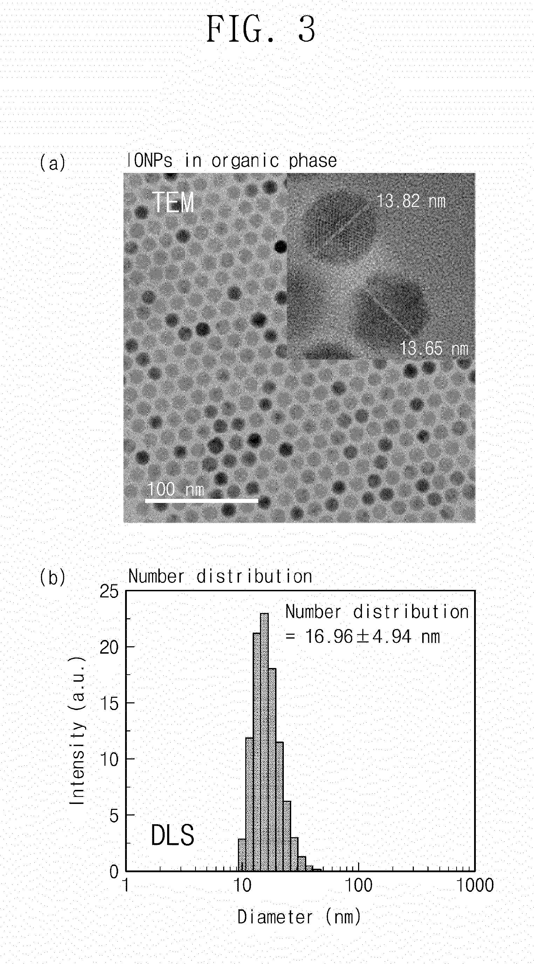

FIG. 3 shows a TEM image and a size analysis result of iron oxide nanoparticles (IONP) dispersed in an organic solvent.

Referring to FIG. 3, a TEM image of the nanoparticle of Example 1 can be seen (a). It is confirmed that the nanoparticle of Example 1 has a very uniform shape and size with an average diameter of about 13 to 14 nm. Since the surface of the nanoparticle of Example 1 is coated with oleic acid, it is confirmed that the nanoparticle is very well dispersed in the organic solvent. However, the nanoparticle of Example 1 has the surface properties which prevent the nanoparticle from being dispersed at all in an aqueous phase.

Furthermore, the size of the nanoparticle of Example 1 dispersed in n-hexane was measured to be about 17 nm by dynamic light scattering (DLS) analysis (b).

### Experimental Example 2: Preparation of an iron oxide nanoparticle coated with indocyanine green.

1 ml of the iron oxide nanoparticle (Example 1) dispersed in n-hexane at a concentration of 10 mg/ml was taken, and then 9 ml of methanol was added thereto to precipitate the iron oxide nanoparticle. Then, supernatant was removed, and 1 ml of chloroform was added thereto to redisperse the iron oxide nanoparticle. After 2 mg of indocyanine green was dissolved in 4 ml of distilled water, 0.1 ml of the iron oxide nanoparticle dispersed in chloroform was added thereto, and tip ultrasonication was performed thereon for 1 minute to prepare a first emulsion solution. The emulsion solution was vigorously stirred for 5 minutes until all the chloroform therein was volatilized and removed

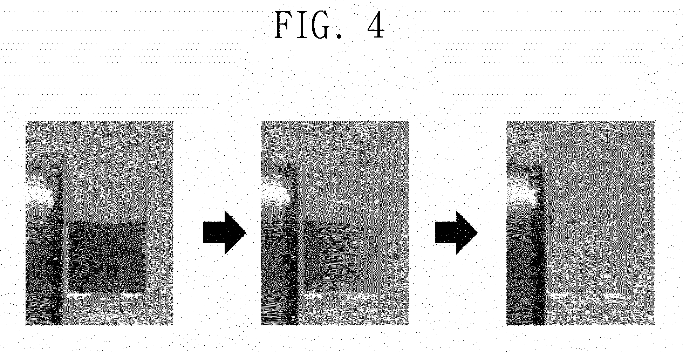

FIG. 4 shows the process of purification using the magnetic force of an iron oxide nanoparticle dispersed in an aqueous phase.

Referring to FIG. 4, the magnetic properties of the iron oxide nanoparticle was used to remove indocyanine green not adsorbed on the iron oxide nanoparticle. Specifically, the solution was held in close contact with a powerful magnet, and when the iron oxide nanoparticle was collected near the magnet, supernatant was removed, and 1 ml of distilled water was added thereto to redisperse the iron oxide nanoparticle. The process was repeated 4-5 times until the indocyanine green in the solution was removed so that the solution became transparent. The finally obtained iron oxide nanoparticle coated with indocyanine green was dispersed in 1 ml of distilled water (Example 2).

### Experimental Example 3: Analysis of the characteristics of an iron oxide nanoparticle coated with indocyanine green

The nanoparticle of Example 2 was dropped on a copper grid coated with carbon to prepare a sample, and a transmission electron microscope (TEM) image thereof was obtained with a high resolution electron microscope of 200 kV (Tecnai F20). Furthermore, the size of the nanoparticle of Example 2 was measured using a particle size surface charge analyzer (Zetasizer Nano-ZS, Otsuka).

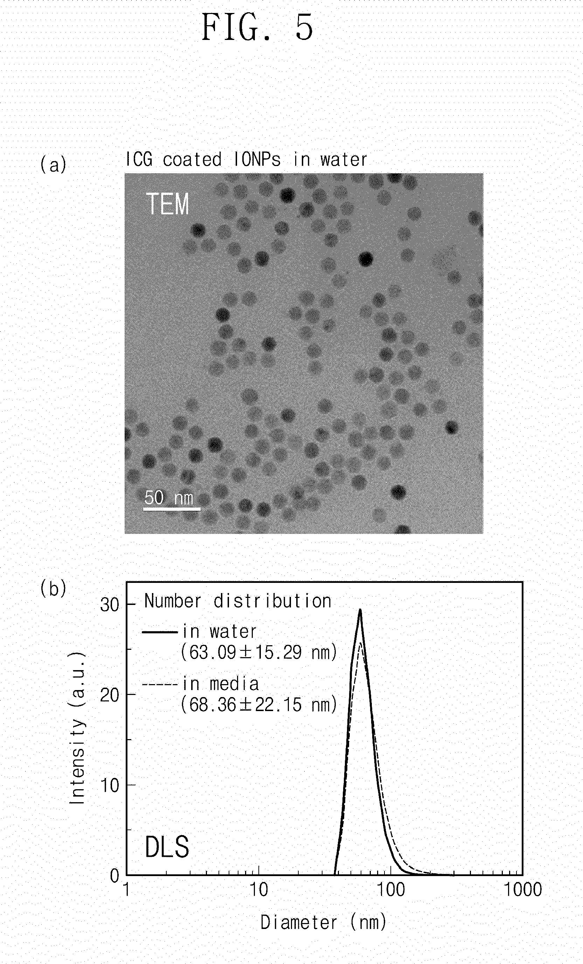

FIG. 5 shows a TEM image and a size analysis result of indocyanine green coated iron oxide nanoparticles (ICG coated IONP) dispersed in an aqueous phase.

Referring to FIG. 5, it was confirmed that the nanoparticle of Example 2, unlike the iron oxide nanoparticle of Example 1, was well dispersed in the aqueous phase without an aggregation phenomenon (a). Due to the indocyanine green which is an amphiphilic organic dye, the surface of the iron oxide nanoparticle was converted to hydrophilic and therefore, the phase conversion thereof was achieved.

The size of the nanoparticle of Example 2 dispersed in the aqueous phase and cell media was measured to be about 63 nm and 69 nm, respectively (b).

The surface charge of the nanoparticle of Example 2 was measured using a particle size surface charge analyzer (Zetasizer Nano-ZS, Otsuka). In addition, the surface charge of a pure indocyanine green solution was measured using the particle size surface charge analyzer.

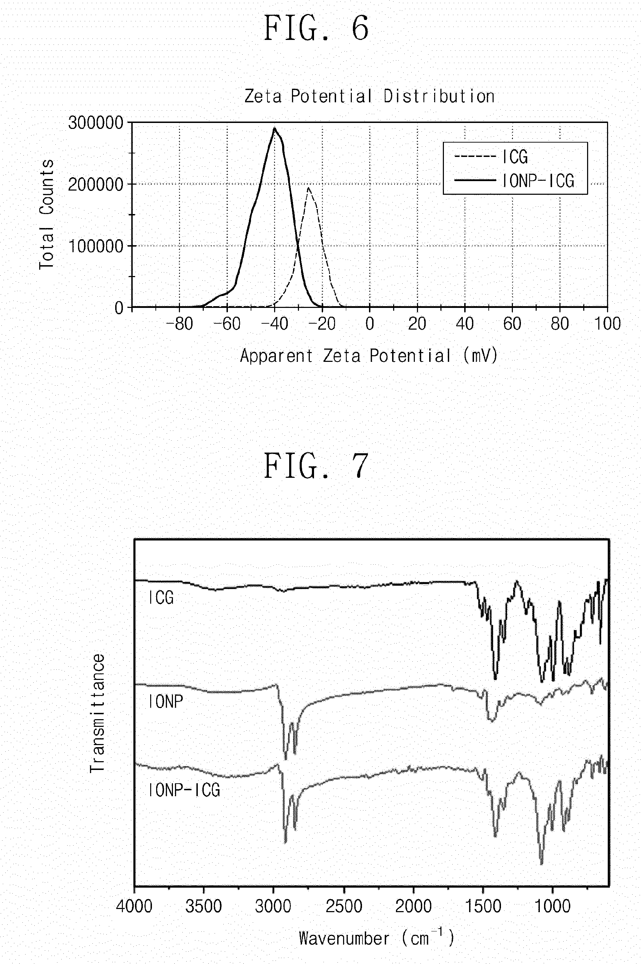

FIG. 6 is a comparative analysis result of the surface charges of an iron oxide nanoparticle coated with indocyanine green (IONP-ICG) and dispersed in an aqueous phase, and a pure indocyanine green (ICG) solution.

When indocyanine green is adsorbed on an iron oxide nanoparticle, a lipophilic group of the indocyanine green is absorbed on the surface of the nanoparticle, so that a hydrophilic group thereof is exposed on an aqueous phase, relatively. Therefore, a charge of the indocyanine green surrounding the nanoparticle may be a charge more negative than that in the pure indocyanine green solution. Referring to FIG. 6, when the surface charges of two solutions were measured and compared, the charge of the pure indocyanine green solution was measured to be -25.1 mV, and the charge of the nanoparticle of Example 2 was measured to be -41.6 mV. Therefore, it is confirmed that the surface charge of the nanoparticle of Example 2 is more negative than that of the pure indocyanine green.

Indocyanine green, the iron oxide nanoparticle of Example 1, and the nanoparticle coated with indocyanine green of Example 2 were prepared, each in a powder state. The FT-IR spectra thereof were measured using a surface reflection infrared spectrometer (ALPHA-P, Bruker). FIG. 7 is a FT-IR analysis result of indocyanine green (ICG), an iron oxide nanoparticle (IONP), and an iron oxide nanoparticle coated with indocyanine green (IONP-ICG).

The absorption and fluorescence spectra of the nanoparticle dispersed in the aqueous phase of Example 2, and the pure indocyanine green were measured using a UV-Vis spectrometer (UV-2600, shimadzu) and a fluorescence spectrometer (FS-2, Sinco). Furthermore, the nanoparticle dispersed in the aqueous phase of Example 2 was prepared in PCR tubes at various concentrations. The T2-weighted MR phantom images thereof were obtained, and each of the 1/T2 values thereof was obtained. Using the obtained 1/T2 values and the concentration ratio, the relaxivity value (r2) was calculated.

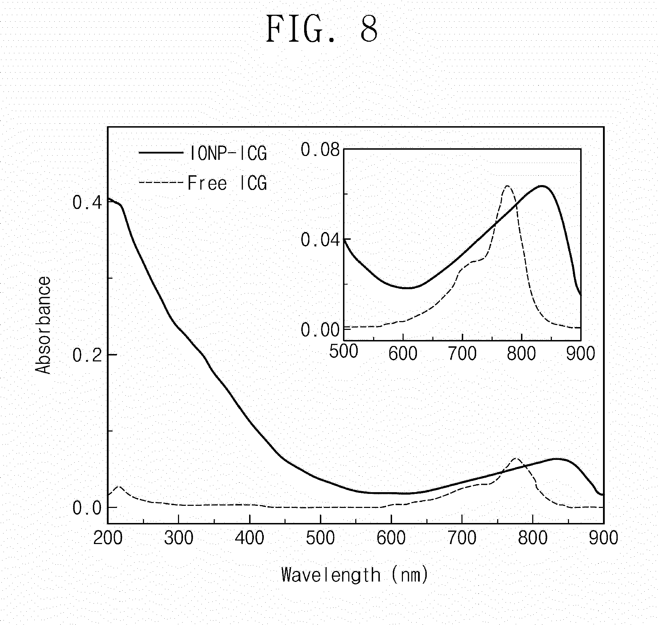

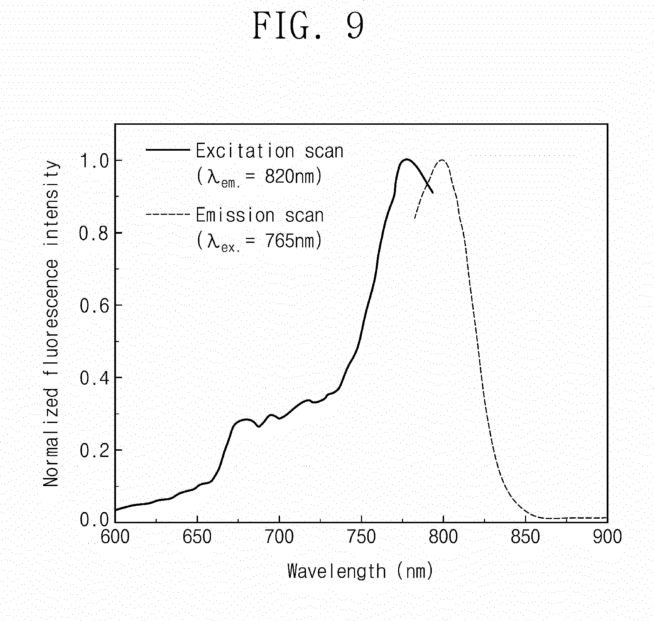

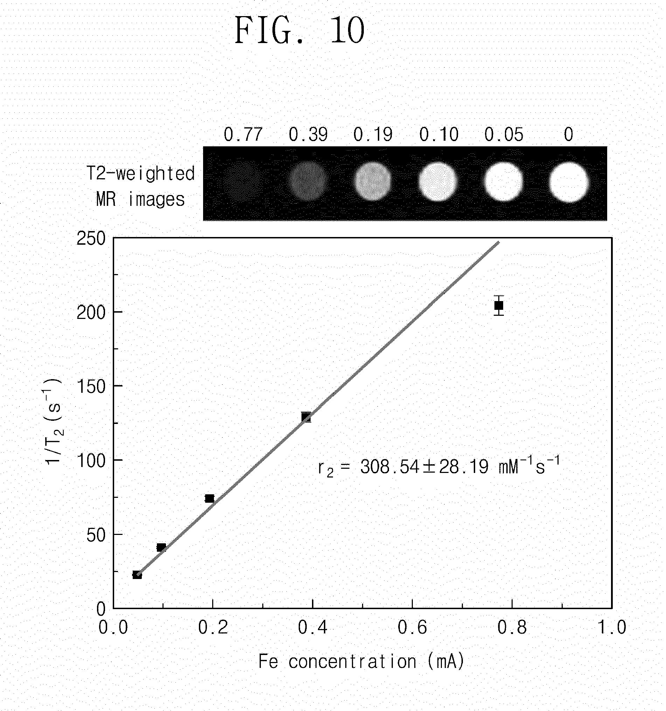

FIGS. 8 and 9 respectively show a comparison analysis result of the absorbance spectrum and fluorescence spectrum of an iron oxide nanoparticle coated with indocyanine green (IONP-ICG) and dispersed in an aqueous phase, and a pure indocyanine green (ICG) solution. FIG. 10 shows T2-weighted MR phantom images and r2 values in accordance with various concentrations of an iron oxide nanoparticle coated with indocyanine green dispersed in an aqueous phase;

Indocyanine green is an amphiphilic dye structurally having both a hydrophilic group and a hydrophobic group, so that, when injected into the blood, indocyanine green is capable of moving exhibiting very good adsorption properties on proteins present in the blood. That is, through a hydrophobic interaction of which a hydrophobic group of indocyanine green is absorbed and embedded on a hydrophobic portion of a protein, the indocyanine green may have good absorption properties. In this case, the absorbance spectrum of indocyanine green may be shifted in a long wavelength direction after being combined with a protein. Referring to FIG. 8, when the absorbance spectra of the nanoparticle of Example 2 and the pure indocyanine green solution were compared, it was confirmed that the absorption wavelength of the nanoparticle of the Example 2 was red-shifted to a long wavelength. That is, a hydrophobic portion of the indocyanine green is adsorbed and coated on the surface of the hydrophobic iron oxide nanoparticle coated with oleic acid.

Referring to FIG. 9, in the case of the fluorescence spectrum of the nanoparticle of Example 2, it is confirmed that, when excited to 765 nm, the near infrared fluorescence signal of 800 nm region is well displayed.

Referring to FIG. 10, it was confirmed the measured relaxivity r2 representing the MR contrasting capability of the nanoparticle of Example 2 was about 308 mM 1s-1. From the result, it is confirmed that the nanoparticle of Example 2 has both characteristics of MR contrasting and near infrared fluorescence contrasting.

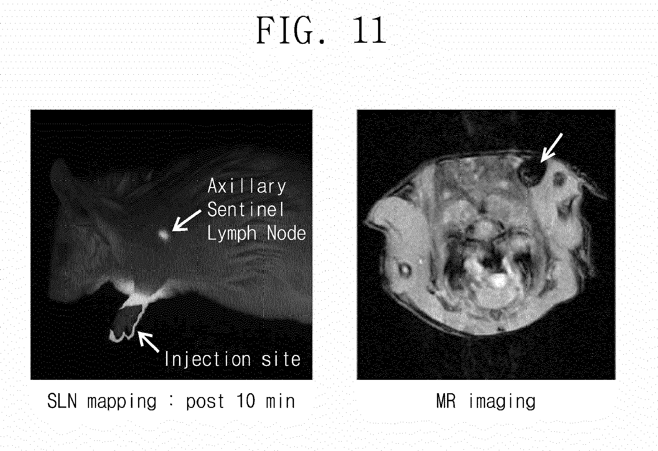

30 µl of the nanoparticle solution of Example 2 was injected into the front sole of a BALB/c mouse. A fluorescence signal obtained by irradiating 808 nm laser to the mouse was captured using an 830 nm long pass filter and an EM-CCD camera. In the same manner, a T2-weighted MR phantom image was obtained using a 4.7 T MRI (Bruker), and an MR image was obtained by cutting a lymph node. The results are shown in FIG. 11. FIG. 11 are an image of fluorescence signal and an MR image thereof, the signal appearing at the lymph node after the injection of iron oxide nanoparticles coated with indocyanine green into a sole a mouse.

### Experimental Example 4: Synthesis of an up-conversion nanoparticle

779.4 mg of yttrium-oleate (Y-oleate, 0.78 mmol), 216.7 mg of ytterbium-oleate (Yb-oleate, 0.20 mmol), 21.6 mg of erbium-oleate (Er-oleate, 0.02 mmol), 8 ml of oleic acid, and 200 g of octadecene (1-octadecene) were mixed. The mixture was stirred for 30 minutes under reduced pressure at room temperature to remove gas and water in the mixture. The mixture was then slowly heated to 100°C for 15 minutes under reduced pressure, and stirred at 100°C for 40 minutes to obtain a reaction solution. The reaction solution was slowly cooled to 50°C under an inert atmosphere, and then 148 mg of ammonium fluoride and 10 ml of methanol in which 100 mg of sodium hydroxide was dissolved were injected into the reaction solution. Thereafter, the reaction solution was stirred at 50°C for 40 minutes under an inert atmosphere. The reaction solution was then slowly heated to 100°C under reduced pressure, and stirred at 100°C for 30 minutes. Thereafter, the reaction solution was slowly heated to 300°C for 1 hour under an inert atmosphere, and stirred at 300°C for 1 hour and 30 minutes. Thereafter, a heater was removed, and the reaction solution was slowly cooled to room temperature, and then 60 ml of ethanol was added thereto to precipitate the formed up-conversion nanoparticle. Then, supernatant was removed, and the up-conversion nanoparticle was redispersed in 1 ml of hexane. 40 ml of ethanol was again added to the up-conversion nanoparticle solution to precipitate the particle, and supernatant was removed to finally obtain an up-conversion nanoparticle (NaYF₄:Yb³⁺,Er³⁺) (Example 3).

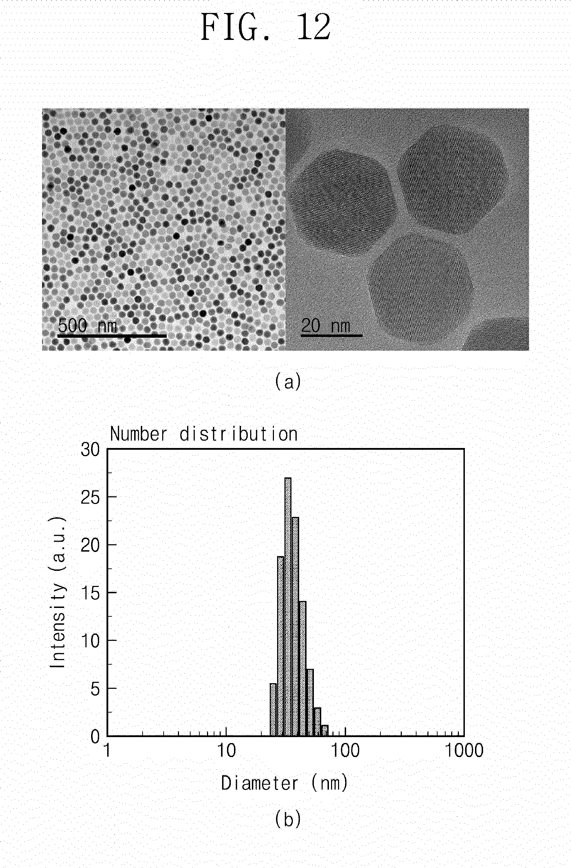

FIG. 12 shows a TEM image and a size analysis result of up-conversion nanoparticles dispersed in an organic solvent.

Referring to FIG. 12, a TEM image of the nanoparticle of Example 3 can be seen (a). Through this, it was confirmed that the nanoparticle of Example 3 has a uniform size and shape. Since the nanoparticle of Example 3 is coated with oleic acid, the nanoparticle may be very well dispersed in the organic solvent. Furthermore, it was confirmed that the size of the nanoparticle of Example 3 has the size distribution of 36.85 ± 9.22 nm according to the result of DLS analysis (b). The up-conversion nanoparticle (NaYF₄:Yb³⁺,Er³⁺) of Example 3 is an optical contrasting agent capable of emitting light in the visible ray area by absorbing a near infrared ray of 980 nm.

### Experimental Example 5: Preparation of an up-conversion nanoparticle coated with indocyanine green

1 mg of the up-conversion nanoparticle (Example 3) was dispersed in 1 ml of chloroform.

After 2 mg of indocyanine green was dissolved in 4 ml of distilled water, 0.1 ml of the nanoparticle of Example 3 dispersed in chloroform was added thereto, and tip ultrasonication was performed thereon for 1 minute to prepare a first emulsion solution. The emulsion solution was vigorously stirred for 5 minutes until all the chloroform therein was volatilized and removed. The stirred solution was centrifuged to precipitate the nanoparticle of Example 3, supernatant was removed, and then distilled water was added thereto again. The process was repeated 4-5 times until the indocyanine green in the solution was removed so that the solution became transparent. The finally obtained up-conversion nanoparticle coated with indocyanine green was dispersed in 1 ml of distilled water (Example 4).

It was confirmed that the nanoparticle of Example 4, unlike the up-conversion nanoparticle of Example 3, was well dispersed in the aqueous phase without an aggregation phenomenon. The phase of the up-conversion nanoparticle was also converted to hydrophilic through the indocyanine green which is an amphiphilic organic dye.

### Example 6: Analysis of the characteristics of an up-conversion nanoparticle coated with indocyanine green

The nanoparticle of Example 4 was spin coated on a slide glass to prepare a sample. 980 nm laser was irradiated on the nanoparticle of Example 4, and the up-conversion PL (photoluminescence) signal in the visible ray area was measured using a 700 nm short pass filter. 785 nm laser was irradiated on the nanoparticle of the Example 4 in in the same position, and the fluorescence signal of the indocyanine green was measured using an 830 nm long pass filter.

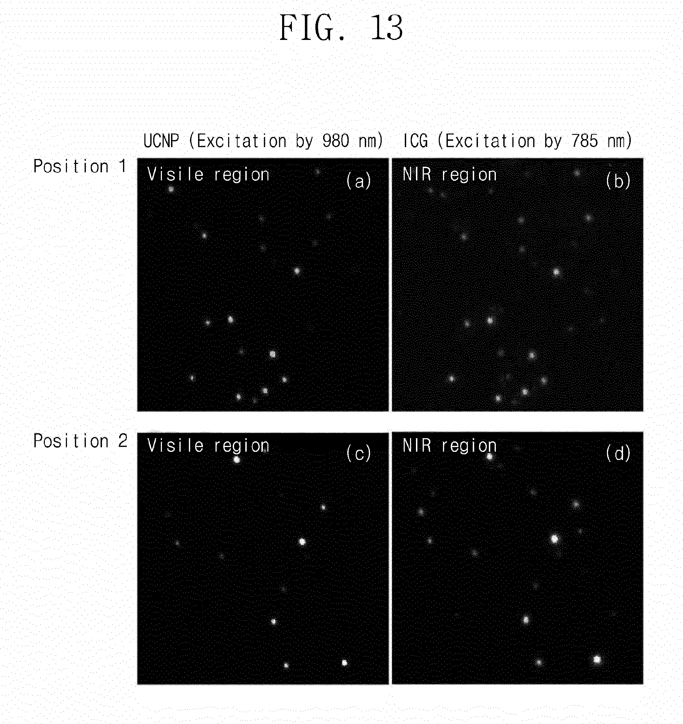

FIG. 13 are photoluminescence (PL) images (UCNP; a, c) of an up-conversion nanoparticle coated with indocyanine green, and fluorescence images (ICG; b, d) of an up-conversion nanoparticle coated with indocyanine green. In FIG. 13, a and b represent the same first position (Position 1), and c and d represent the same second position (Position 2).

Referring to FIG. 13, both the PL and fluorescence signals of the nanoparticle of Example 4 are well displayed. Through this, it was confirmed that indocyanine green coating was well formed on the surface of the nanoparticle of Example 4. Also, in the case of the nanoparticle of Example 4, a dual color optical contrasting may be possible through a combination of two optical contrasting agents (up-conversion, and fluorescence). That is, selective observation at two specific wavelengths may be possible using a single particle.

A slide glass on which the nanoparticle of Example 4 was spin coated thereon was prepared. A region of the slide glass was selected, and was continuously irradiated by 785 nm laser for 30 minutes.

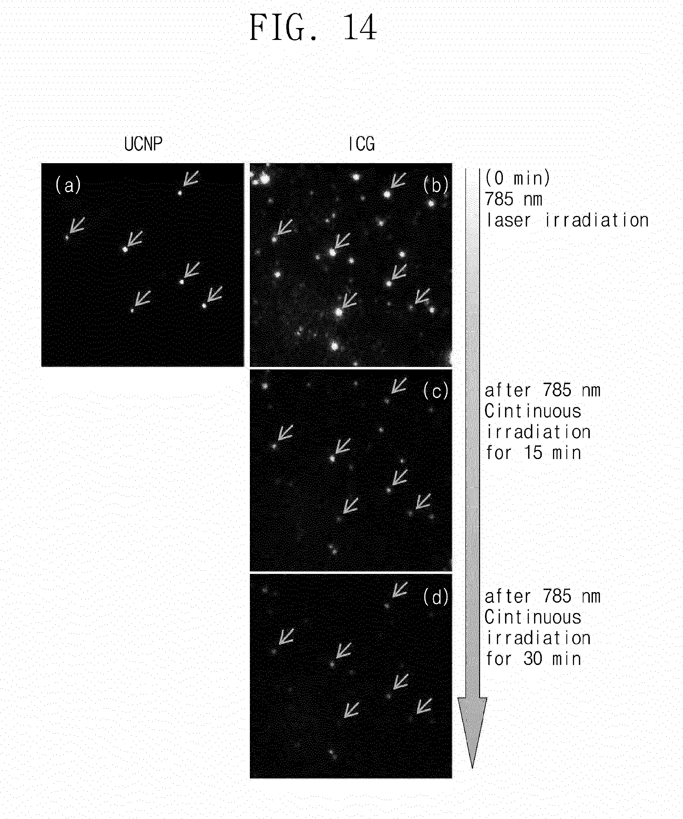

FIG. 14 is a single particle fluorescence image showing the optical stability of an up-conversion nanoparticle coated with indocyanine green. In FIG. 14, a is an up-conversion signal, and b to d in FIG. 4 are fluorescence signals of indocyanine green over time

Referring to FIG. 14, when the up-conversion PL signal (a) and the fluorescence signals (b-d) over time were compared, it was confirmed that the fluorescence signal of indocyanine green continued to appear even after 30 minutes of laser irradiation in the positions on which up-conversion nanoparticles are present (yellow arrows). On the other hand, in the position on which nanoparticles are not present, it was confirmed that the fluorescence signal of indocyanine green were relatively much reduced or disappeared after 30 minutes. This may indicate that the optical stability is increased by coating the indocyanine green on the nanoparticle. As described above, the hydrophobic portion of indocyanine green interacts with the surface of the nanoparticle and is combined therewith, thereby being in a structurally bound (non-flexible) state. Such a state may reduce the photobleaching phenomenon due to a structural change of the indocyanine green, so that optical stability may be increased.

The inventive concept may provide a new aspect of phase conversion method in that an amphiphilic organic dye is used as interface material which is a medium for phase conversion. Furthermore, the utilization as a fluorescence contrasting agent may be great in that the fluorescence stability of the amphiphilic organic dyes adsorbed on the surface of the particle may be increased.

## Claims

1. A hydrophilic nanoparticle comprising:
a hydrophobic particle comprising a center particle, and a hydrophobic ligand covering a surface of the center particle; and
an amphiphilic organic dye combined with the hydrophobic ligand by a hydrophobic interaction, and
wherein the hydrophilic particle has a surface zeta potential lower than a surface zeta potential of the amphiphilic organic dye, **characterized in that**:
the center particle comprises a transition metal oxide or is an up-conversion particle;
the hydrophobic ligand comprises a fatty acid;
the amphiphilic organic dye is selected from the group consisting of cyanine; and
the hydrophilic particle is obtainable from a process for manufacturing a hydrophilic particle comprising the steps of:
preparing said hydrophobic particle dispersed in an organic phase; and
mixing the hydrophobic particle in the organic phase with said amphiphilic organic dye in an aqueous phase to form said hydrophilic particle,
wherein the amphiphilic organic dye is directly adsorbed on a surface of the hydrophobic particle to phase-convert the hydrophobic particle to the hydrophilic particle dispersed in the aqueous phase.

2. The hydrophilic nanoparticle of claim 1, wherein the center particle comprises a transition metal oxide, and
wherein the transition metal oxide is optionally selected from the group consisting of iron oxide, manganese oxide, titanium oxide, nickel oxide, cobalt oxide, zinc oxide, ceria, and gadolinium oxide; and
wherein the fatty acid is optionally selected from the group consisting of oleic acid, laurate acid, palmitic acid, linoleic acid, and stearic acid.

3. The hydrophilic nanoparticle of claim 1, wherein the up-conversion particle is selected from the group consisting of NaYF₄:Yb³⁺,Er³⁺, NaYF₄:Yb³⁺,Tm³⁺, NaGdF₄:Yb³⁺,Er³⁺, NaGdF₄:Yb³⁺,Tm³⁺, NaYF₄:Yb³⁺,Er³⁺/NaGdF₄, NaYF₄:Yb³⁺,Tm³⁺/NaGdF₄, NaGdF₄:Yb³⁺,Tm³⁺/NaGdF₄, and NaGdF₄:Yb³⁺,Er³⁺/NaGdF₄.

4. The hydrophilic nanoparticle of claim 1, wherein the fatty acid is selected from the group consisting of oleic acid, laurate acid, palmitic acid, linoleic acid, and stearic acid.

5. The hydrophilic nanoparticle of claim 1, wherein the surface zeta potential of the amphiphilic organic dye is a value measured when the amphiphilic organic dye is present alone, and/or
wherein the surface zeta potential of the hydrophilic particle is a negative charge.

6. The hydrophilic nanoparticle of claim 1, wherein an average diameter of the hydrophilic particle is greater than an average diameter of the hydrophobic particle.

7. A method for manufacturing a hydrophilic nanoparticle comprising:
preparing a hydrophobic particle dispersed in an organic phase; and
mixing the hydrophobic particle in the organic phase with an amphiphilic organic dye in an aqueous phase to form a hydrophilic particle,
wherein the amphiphilic organic dye is directly adsorbed on a surface of the hydrophobic particle to phase-convert the hydrophobic particle to the hydrophilic particle dispersed in the aqueous phase, and
wherein the hydrophobic particle comprises a center particle, and a hydrophobic ligand covering a surface of the center particle, **characterized in that**:
the center particle comprises a transition metal oxide or is an up-conversion particle;
the hydrophobic ligand comprises a fatty acid; and
the amphiphilic organic dye is selected from the group consisting of cyanine.

8. The method of claim 7, wherein the mixing of the hydrophobic particle and the amphiphilic organic dye comprises:
adding the hydrophobic particle in the organic phase to the amphiphilic organic dye in the aqueous phase; and
ultrasonicating a mixture of the hydrophobic particle and the amphiphilic organic dye to form a water-in-oil (O/W) emulsion.

9. The method of claim 7, wherein the organic phase comprises an organic solvent selected from the group consisting of chloroform, cyclohexane, hexane, heptane, octane, isooctane, nonane, decane, and toluene.

10. The method of claim 7, wherein the hydrophobic particle comprises a hydrophobic ligand on a surface thereof, and
wherein the amphiphilic organic dye is combined to the hydrophobic ligand by a hydrophobic interaction.

11. The method of claim 7 further comprising, after forming the hydrophilic particle, evaporating the organic solvent forming the organic phase.

12. A contrasting agent comprising a hydrophilic nanoparticle according to any one of Claims 1 to 6.

13. The contrasting agent of claim 12 2. for use in magnetic resonance imaging, optical imaging, or magnetic resonance imaging and optical imaging.

## Patentansprüche

1. Hydrophiles Nanopartikel, umfassend:
ein hydrophobes Partikel, das ein zentrales Partikel umfasst, und einen hydrophoben Liganden, der eine Oberfläche des zentralen Partikels bedeckt; und
einen amphiphilen organischen Farbstoff, der mit dem hydrophoben Liganden durch eine hydrophobe Wechselwirkung vereint ist, und
wobei das hydrophile Partikel ein Oberflächenzetapotential aufweist, das geringer ist als ein Oberflächenzetapotential des amphiphilen organischen Farbstoffes,
**dadurch gekennzeichnet, dass**:
das zentrale Partikel ein Übergangsmetalloxid umfasst oder ein hochkonvertiertes Partikel ist;
der hydrophobe Ligand eine Fettsäure umfasst;
der amphiphile organische Farbstoff ausgewählt ist aus der Gruppe bestehend aus Cyanin; und
das hydrophile Partikel erhalten wird durch ein Verfahren zum Herstellen eines hydrophilen Partikels, das folgende Schritte umfasst:
Vorbereiten des hydrophoben Partikels, das in einer organischen Phase dispergiert ist; und
Mischen des hydrophoben Partikels in der organischen Phase mit dem amphiphilen organischen Farbstoff in einer wässrigen Phase, um das hydrophile Partikel zu bilden,
wobei der amphiphile organische Farbstoff direkt auf einer Oberfläche des hydrophoben Partikels adsorbiert ist, um das hydrophobe Partikel einer Phasenumwandlung zu dem hydrophilen Partikel, das in der wässrigen Phase dispergiert ist, zu unterziehen.

2. Hydrophiles Nanopartikel nach Anspruch 1, wobei das zentrale Partikel ein Übergangsmetalloxid umfasst, und
wobei das Übergangsmetalloxid gegebenenfalls ausgewählt ist aus der Gruppe bestehend aus Eisenoxid, Manganoxid, Titanoxid, Nickeloxid, Kobaltoxid, Zinkoxid, Ceroxid und Gadoliniumoxid; und
wobei die Fettsäure gegebenenfalls ausgewählt ist aus der Gruppe bestehend aus Ölsäure, Laurinsäure, Palmitinsäure, Linolsäure und Stearinsäure.

3. Hydrophiles Nanopartikel nach Anspruch 1, wobei das hochkonvertierte Partikel ausgewählt ist aus der Gruppe bestehend aus NaYF₄:Yb³⁺,Er³⁺, NaYF₄ : Yb³⁺,Tm³⁺, NaGdF₄ : Yb³⁺,Er³⁺, NaGdF₄ : Yb³⁺,Tm³⁺, NaYF₄ : Yb³⁺,Er³⁺/NaGdF₄,NaY F₄ : Yb³⁺, Tm³+lNaGd F₄, NaGdF₄ : Yb³⁺, Tm³⁺/NaGdF₄ und NaGdF₄ : Yb³⁺, Er³⁺/NaGdF₄.

4. Hydrophiles Nanopartikel nach Anspruch 1, wobei die Fettsäure ausgewählt ist aus der Gruppe bestehend aus Ölsäure, Laurinsäure, Palmitinsäure, Linolsäure und Stearinsäure.

5. Hydrophiles Nanopartikel nach Anspruch 1, wobei das Oberflächenzetapotential des amphiphilen organischen Farbstoffs ein Wert ist, der gemessen wird, wenn der amphiphile organische Farbstoff allein vorhanden ist, und/oder
wobei das Oberflächenzetapotential des hydrophilen Partikels eine negative Ladung ist.

6. Hydrophiles Nanopartikel nach Anspruch 1, wobei ein mittlerer Durchmesser des hydrophilen Partikels grösser ist als ein mittlerer Durchmesser des hydrophoben Partikels.

7. Verfahren zum Herstellen eines hydrophilen Nanopartikels, umfassend:
Vorbereiten eines hydrophoben Partikels, das in einer organischen Phase dispergiert ist; und
Mischen des hydrophoben Partikels in der organischen Phase mit einem amphiphilen organischen Farbstoff in einer wässrigen Phase, um ein hydrophiles Partikel zu bilden,
wobei der amphiphile organische Farbstoff direkt auf einer Oberfläche des hydrophoben Partikels adsorbiert wird, um das hydrophobe Partikel einer Phasenumwandlung zu dem hydrophilen Partikel, das in der wässrigen Phase dispergiert ist, zu unterziehen,
wobei das hydrophobe Partikel ein zentrales Partikel und einen hydrophoben Liganden umfasst, der eine Oberfläche des zentralen Partikels bedeckt,
**dadurch gekennzeichnet, dass**:
das zentrale Partikel ein Übergangsmetalloxid umfasst oder ein hochkonvertiertes Partikel ist;
der hydrophobe Ligand eine Fettsäure umfasst; und
der amphiphile organische Farbstoff ausgewählt ist aus der Gruppe bestehend aus Cyanin.

8. Verfahren nach Anspruch 7, wobei das Mischen des hydrophoben Partikels und des amphiphilen organischen Farbstoffs umfasst:
Zugeben des hydrophoben Partikels in der organischen Phase zu dem amphiphilen organischen Farbstoff in der wässrigen Phase; und
Ultraschallbehandeln einer Mischung aus dem hydrophoben Partikel und dem amphiphilen organischen Farbstoff, um eine Wasser-in-Öl (O/W)-Emulsion zu bilden.

9. Verfahren nach Anspruch 7, wobei die organische Phase ein organisches Lösemittel umfasst, das ausgewählt ist aus der Gruppe bestehend aus Chloroform, Cyclohexan, Hexan, Heptan, Octan, Isooctan, Nonan, Decan und Toluol.

10. Verfahren nach Anspruch 7, wobei das hydrophobe Partikel einen hydrophoben Liganden auf einer Oberfläche davon umfasst, und
wobei der amphiphile organische Farbstoff durch eine hydrophobe Wechselwirkung mit dem hydrophoben Liganden vereint wird.

11. Verfahren nach Anspruch 7, ferner umfassend: nach Bilden des hydrophilen Partikels Verdampfen des organischen Lösemittels, das die organische Phase bildet.

12. Kontrastmittel, umfassend ein hydrophiles Nanopartikel nach einem der Ansprüche 1 bis 6.

13. Kontrastmittel nach Anspruch 12 zur Verwendung in der Bildgebung durch Magnetresonanz, der optischen Bildgebung oder der Bildgebung durch Magnetresonanz und der optischen Bildgebung.

## Revendications

1. Nanoparticule hydrophile comprenant :
une particule hydrophobe comprenant une particule centrale et un ligand hydrophobe recouvrant une surface de la particule centrale, et
un colorant organique amphiphile combiné au ligand hydrophobe par une interaction hydrophobe, et
dans laquelle la particule hydrophile a un potentiel zêta de surface inférieur au potentiel zêta de surface du colorant organique amphiphile,
**caractérisée en ce que** :
la particule centrale comprend un oxyde de métal de transition ou est une particule à conversion ascendante,
le ligand hydrophobe comprend un acide gras,
le colorant organique amphiphile est sélectionné dans le groupe comprenant les cyanines, et
la particule hydrophile peut être obtenue par un procédé de fabrication d'une particule hydrophile comprenant les étapes consistant à :
préparer ladite particule hydrophobe dispersée dans une phase organique, et
mélanger la particule hydrophobe dans la phase organique avec ledit colorant amphiphile organique dans une phase aqueuse pour former ladite particule hydrophile,
dans laquelle le colorant organique amphiphile est directement adsorbé sur une surface de la particule hydrophobe pour transformer en phase la particule hydrophobe en la particule hydrophile dispersée dans la phase aqueuse.

2. Nanoparticule hydrophile selon la revendication 1, dans laquelle la particule centrale comprend un oxyde de métal de transition, et
dans laquelle l'oxyde de métal de transition est facultativement sélectionné dans le groupe comprenant l'oxyde de fer, l'oxyde de manganèse, l'oxyde de titane, l'oxyde de nickel, l'oxyde de cobalt, l'oxyde de zinc, l'oxyde de cérium et l'oxyde de gadolinium, et
dans laquelle l'acide gras est facultativement sélectionné dans le groupe comprenant l'acide oléique, l'acide laurique, l'acide palmitique, l'acide linoléique et l'acide stéarique.

3. Nanoparticule hydrophile selon la revendication 1, dans laquelle la particule à conversion ascendante est sélectionnée dans le groupe comprenant NaYF₄ : Yb³⁺,Er³⁺, NaYF₄ : Yb³⁺,Tm³⁺, NaGdF₄ : Yb³⁺,Er³⁺, NaGdF₄ : Yb³⁺,Tm³⁺, NaYF₄ : Yb³⁺,Er³⁺/ NaGdF₄, NaYF₄: Yb³⁺,Tm³⁺/ NaGdF₄, NaGdF₄ : Yb³⁺,Tm³⁺/ NaGdF₄ et NaGdF₄: Yb³⁺,Er³⁺/ NaGdF₄.

4. Nanoparticule hydrophile selon la revendication 1, dans laquelle l'acide gras est sélectionné dans le groupe comprenant l'acide oléique, l'acide laurique, l'acide palmitique, l'acide linoléique et l'acide stéarique.

5. Nanoparticule hydrophile selon la revendication 1, dans laquelle le potentiel zêta de surface du colorant organique amphiphile est une valeur mesurée lorsque le colorant organique amphiphile est présent seul, et/ou
dans laquelle le potentiel zêta de surface de la particule hydrophile est une charge négative.

6. Nanoparticule hydrophile selon la revendication 1, dans laquelle le diamètre moyen de la particule hydrophile est supérieur au diamètre moyen de la particule hydrophobe.

7. Procédé de fabrication d'une nanoparticule hydrophile comprenant les étapes consistant à :
préparer une particule hydrophobe dispersée dans une phase organique, et
mélanger la particule hydrophobe dans la phase organique avec un colorant organique amphiphile dans une phase aqueuse pour former une particule hydrophile,
dans lequel le colorant organique amphiphile est directement adsorbé sur une surface de la particule hydrophobe pour transformer en phase la particule hydrophobe en la particule hydrophile dispersée dans la phase aqueuse, et
dans lequel la particule hydrophobe comprend une particule centrale et un ligand hydrophobe recouvrant une surface de la particule centrale,
**caractérisé en ce que** :
la particule centrale comprend un oxyde de métal de transition ou est une particule à conversion ascendante,
le ligand hydrophobe comprend un acide gras, et
le colorant amphiphile organique est sélectionné dans le groupe comprenant les cyanines.

8. Procédé selon la revendication 7, dans lequel le mélange de la particule hydrophobe et du colorant organique amphiphile comprend :
l'ajout de la particule hydrophobe dans la phase organique au colorant organique amphiphile dans la phase aqueuse, et
le traitement aux ultrasons d'un mélange de la particule hydrophobe et du colorant organique amphiphile pour former une émulsion eau-dans-huile (H/E).

9. Procédé selon la revendication 7, dans lequel la phase organique comprend un solvant organique sélectionné dans le groupe comprenant le chloroforme, le cyclohexane, l'hexane, l'heptane, l'octane, l'isooctane, le nonane, le décane et le toluène.

10. Procédé selon la revendication 7, dans lequel la particule hydrophobe comprend un ligand hydrophobe sur une surface de celle-ci, et
dans lequel le colorant organique amphiphile est combiné au ligand hydrophobe par une interaction hydrophobe.

11. Procédé selon la revendication 7, comprenant en outre, après la formation de la particule hydrophile, une évaporation du solvant formant la phase organique.

12. Agent de contraste comprenant une nanoparticule hydrophile selon l'une quelconque des revendications 1 à 6.

13. Agent de contraste selon la revendication 12 pour une utilisation dans l'imagerie par résonance magnétique, l'imagerie optique ou l'imagerie par résonance magnétique et l'imagerie optique.
